# FASCICULE DE BREVET EUROPEEN

(11) **EP 1 773 420 B1**
(45) Date de publication et mention de la délivrance du brevet: **08.09.2010**
(21) Numéro de dépôt: 05786212.0
(22) Date de dépôt: 01.07.2005
(51) Int. Cl.: A61L 27/38, A61K 35/37

(54) **UTILISATION DE FIBROBLASTES GINGIVAUX EN THERAPIE CELLULAIRE VASCULAIRE.**
VERWENDUNG VON ZAHNFLEISCHFIBROBLASTEN ZUR BEHANDLUNG VON VASKULÄREN ZELLEN
USE OF GINGIVAL FIBROBLASTS FOR VASCULAR CELL THERAPY

(30) Priorité: 02.07.2004 FR 0407357
(43) Date de publication de la demande: 18.04.2007
(73) Titulaire: UNIVERSITE PARIS DESCARTES, 75270 Paris Cedex 06 (FR); Assistance Publique - Hôpitaux de Paris, 75184 Paris Cedex 04 (FR)
(72) Inventeur: LAFONT, Antoine, F-75007 PARIS (FR); GOGLY, Bruno, F-77510 HONDEVILLIERS (FR)
(74) Mandataire: Vialle-Presles, Marie José
(86) Numéro de dépôt international: PCT/FR2005/001690
(87) Numéro de publication internationale: WO 2006/013261

(56) Documents cités:
- EP-A- 0 393 788
- WO-A-01/82773
- WO-A-98/22154
- WO-A-99/51164
- US-A- 5 885 829

## Description

La présente invention est relative à l'utilisation de fibroblastes gingivaux en thérapie cellulaire vasculaire, notamment pour le traitement de pathologies artérielles.

La paroi artérielle est constituée de trois couches superposées, dénommées tuniques. La tunique interne (intima) est constituée d'une couche de cellules endothéliales. La tunique intermédiaire (média) est principalement constituée de fibres musculaires lisses et de tissu conjonctif riche en fibres élastiques. La tunique externe (adventice) est une enveloppe conjonctive qui entoure l'ensemble.

Les artères sont soumises à de nombreuses agressions d'origine diverse (hypoxie, surcharge lipidique, forces hémodynamiques, athérome, hypertension, etc.), pouvant induire des lésions de la paroi artérielle. Lors de la réparation de ces lésions, peut intervenir une réaction de cicatrisation anormale, résultant d'un déséquilibre entre la dégradation et la synthèse de la matrice extracellulaire, et induisant un remodelage artériel pathologique, qui peut se traduire par un élargissement vasculaire (anévrisme), ou au contraire par une constriction (sténose, survenant secondairement au cours de l'athérogénèse, ou resténose, consécutive notamment à une angioplastie évoluant au décours du remodelage cicatriciel vers la réapparition d'une sténose).

Par exemple, dans les lésions anévrismales, on observe, au niveau de la media, une dégradation enzymatique des composants de la matrice extracellulaire, notamment des fibres élastiques, et une diminution du nombre de cellules musculaires lisses (CML) ; au niveau de l'adventice on observe une fibrose accompagnée d'une importante infiltration inflammatoire.

La dégradation des composants de la matrice extracellulaire implique diverses protéases matricielles. Parmi celles-ci on citera notamment une élastase, la métalloprotéinase-9 matricielle (MMP9) synthétisée par les leucocytes infiltrés et les cellules hôtes physiologiques (cellules endothéliales, cellules musculaires lisses et fibroblastes adventiciels) et qui est en grande partie responsable de la fragmentation des fibres élastiques (THOMPSON, J. Clin. Invest. 96 : 318-326, 1995). Parallèlement, la raréfaction des CMLs dans la media entraîne une diminution de la synthèse des composants de la matrice extracellulaire, et de celle des inhibiteurs des protéases matricielles, qui sont principalement synthétisés par ces CMLs (LOPEZ-CANDALES et al., Am. J. Pathol. 150 : 993-1007, 1997).

Dans le cas de la sténose, et de la resténose intervient un remodelage fibreux rétractile qui peut également être considéré comme un processus de cicatrisation anormal. Ce remodelage se caractérise par une hyperplasie fibreuse intimale (LAFONT et al., Circ. Res. 76(6) : 996-1002, 1995), et apparaît relié à une augmentation du collagène au niveau de la néointima, de la média et de l'adventice (LAFONT et al., Circulation 100(10) : 1109-1115, 1999 ; DURAND et al., Arch. Mal. Coeur Vaiss. 94(6) : 605-611, 2001).

A l'heure actuelle, le traitement des pathologies artérielles repose principalement sur la chirurgie et la cardiologie interventionnelle (chirurgie vasculaire, pontages aorto-coronaires et périphériques, prothèse aortiques en dacron, angioplastie artérielle coronaire et périphérique, endoprothèses aortiques, etc...). Cependant, ces techniques invasives ne traitent pas la cause mais les conséquences de la pathologie. Elles permettent une amélioration de la situation du patient sans pouvoir enrayer le développement de la maladie.

Il apparaît donc nécessaire de disposer de nouveaux moyens de traitement moins invasifs, et permettant de traiter efficacement le remodelage artériel pathologique.

ALLAIRE et al. (J. Clin. Invest. 102(7) : 1413-1420, 1998) ont observé, dans un modèle d'anévrisme expérimental chez le rat, que l'implantation locale de CMLs dans la paroi artérielle induisait une inhibition de la dégradation des fibres élastiques et de la formation d'un anévrisme. Il a également été montré chez le rat que des anévrismes expérimentaux déjà formés peuvent être stabilisés par injection locale de CML. Cette stabilisation est associée à une sécrétion de TGFβ d'origine paracrine au niveau de la transplantation cellulaire (LOSY et al., J. Vasc. Surg. 37(6) : 1301-1309, 2003).

Cependant, un obstacle majeur à l'utilisation de CMLs pour le traitement des anévrismes par thérapie cellulaire provient de la nécessité d'obtenir ces CMLs à partir de cellules souches. Les cellules souches utilisées proviennent de la moelle ou du sang et sont très difficiles à obtenir.

Les Inventeurs ont entrepris de rechercher si d'autres types de cellules que les CML de la paroi artérielle pouvaient éventuellement être utilisées en thérapie cellulaire, et ont eu l'idée de tester les fibroblastes gingivaux.

Les fibroblastes gingivaux sont des cellules mésenchymateuses capables de migrer, d'adhérer, et de proliférer au sein des tissus conjonctifs mous de la gencive, maintenant ainsi l'intégrité du tissu gingival soumis à de nombreuses agressions telles que les forces mécaniques, les infections bactériennes, les variations de pH, de température, etc... (GOGLY et al., Clin. Oral Invest. 1 : 1 147-152, 1997 ; GOGLY et al., Biochem. Pharmacol. 56(11) : 1447-1454, 1998 ; EJEIL et al., J. Periodontol. 74(2) : 188-195, 2003).

Selon les conditions environnementales auxquelles ils sont soumis, les fibroblastes gingivaux sont capables de changer de phénotype, et de répondre aux stimuli provenant de l'environnement tissulaire gingival par prolifération, migration, synthèse ou dégradation des composants de la matrice extracellulaire.

Ils sont ainsi capables de synthétiser divers composants de la matrice extracellulaire : des collagènes (types I, III, V, VI, VII, XII), des fibres élastiques, des protéoglycanes et glycosaminoglycanes, des glycoprotéines. Ils peuvent produire aussi une variété d'enzymes, (notamment métalloprotéases), capables de dégrader ces composants macromoléculaires. Enfin ils peuvent exprimer également des inhibiteurs tissulaires des métalloprotéases, qui inhibent les formes actives des MMPs.

Les Inventeurs ont effectué des co-cultures de fibroblastes gingivaux et de CMLs, et ont observé que les interactions entre ces cellules conduisaient à l'inhibition par les fibroblastes gingivaux de l'activité de MMP9 par les CML, ainsi qu'à la potentialisation de la sécrétion de TGFβ par les deux types cellulaires. Ces effets sont spécifiques des fibroblastes gingivaux, et ne sont pas observés lorsque les CMLs sont co-cultivées avec des fibroblastes dermiques ou adventiciels.

Des observations identiques ont été effectuées dans des co-cultures de fibroblastes gingivaux et d'artères lésées. Ce modèle organotypique a également permis de montrer que la présence des fibroblastes gingivaux induit une protection du réseau élastique.

Il apparaît donc que les fibroblastes gingivaux ont d'une part un effet inhibiteur sur l'un des facteurs principaux de la formation des anévrismes, à savoir la MMP9 responsable de la dégradation du réseau élastique, et d'autre part un effet activateur sur un facteur favorable à la stabilisation des anévrismes, à savoir la sécrétion de TGFβ.

Les Inventeurs ont en outre effectué, chez le lapin, des essais *in vivo* de transplantation de fibroblastes gingivaux obtenus à partir d'un prélèvement de tissu gingival d'un lapin, dans la paroi artérielle dudit lapin, et ont constaté que ces fibroblastes gingivaux autologues étaient capables de s'insérer dans la paroi, au niveau de la media.

La présente invention a donc pour objet l'utilisation de fibroblastes gingivaux pour l'obtention d'une composition cellulaire destinée au traitement d'une pathologie du remodelage artériel.

Les fibroblastes gingivaux peuvent être utilisés, conformément à l'invention, dans toutes les pathologies vasculaires, notamment artérielles, avec altérations profondes de la matrice extracellulaire. On citera en particulier : l'athérosclérose, la plaque vulnérable, la resténose, les anévrismes artériels, la dissection aortique.

Selon un mode de mise en oeuvre préféré de la présente invention, ladite pathologie est un anévrisme.

Selon un autre mode de mise en oeuvre préféré de la présente invention, ladite pathologie est une sténose ou une resténose après angioplastie.

Lesdits fibroblastes sont de préférence des fibroblastes autologues, c'est à dire des fibroblastes issus de tissu gingival préalablement prélevé chez le sujet auquel est destiné le traitement, et mis en culture. De préférence ces fibroblastes sont cultivés pendant au moins 14 jours, et avantageusement pendant 14 à 70 jours.

Des cultures de fibroblastes gingivaux utilisables pour la mise en oeuvre de la présente invention peuvent être obtenues par techniques classiques, qui sont connues en elles-mêmes de l'homme du métier (BARLOVATZ-MEIMON et al., « culture de cellules animales » p.898, ill. Paris : INSERM, 2003).

L'implantation des fibroblastes dans la paroi artérielle peut s'effectuer de diverses façons ; soit localement par injection à l'aide de cathéters à ballonnets munis de micro-aiguilles (INFILTRATOR) injection par voie externe ou interne dans l'adventice ou le tissu périadventitiel, soit par voie générale (veineuse périphérique ou artérielle en amont du site d'injection avec adressage réalisé par un guidage magnétique (incorporation intracellulaire préalable de nanoparticules superparamagnétiques), ou biologique (WILHEM et al., Eur. Biophys. J. 31(2) : 118-125, 2002 ; PANYAM et al., J. Drug Target 10(6): 515-523, 2002).

On peut également, si on le souhaite, associer aux fibroblates gingivaux des produits intervenant dans le remodelage matriciel, par exemple la décorine (protéoglycanne intervenant dans le remodelage matriciel) (AL HAJ ZEN et al, Matrix Biol. 22(3) : 251-258, 2003) ou l'acide hyaluronique.

La présente invention sera mieux comprise à l'aide du complément de description qui va suivre, qui se réfère à des exemples non-limitatifs illustrant des effets des fibroblastes gingivaux utilisables en thérapie cellulaire vasculaire.

### EXEMPLE 1 : SECRETION DE MMP2, MMP9, TIMP-1 ET TGFβ DANS DES CO-CULTURES DE FIBROBLASTES GINGIVAUX ET DE CELLULES MUSCULAIRES LISSES

### Marquage des fibroblastes

Les fibroblastes gingivaux sont marqués par des nanoparticules anioniques de maghémite comme décrit par WILHEM et al. (Biomaterials 24 : 1001-1011, 2003).

Les fibroblastes gingivaux (six cultures différentes) sont cultivés jusqu'à confluence dans du sérum de veau foetal (SVF) 10% ; et après 48 h, le surnageant de culture est prélevé, et les cellules sont cultivées dans du milieu sans sérum.

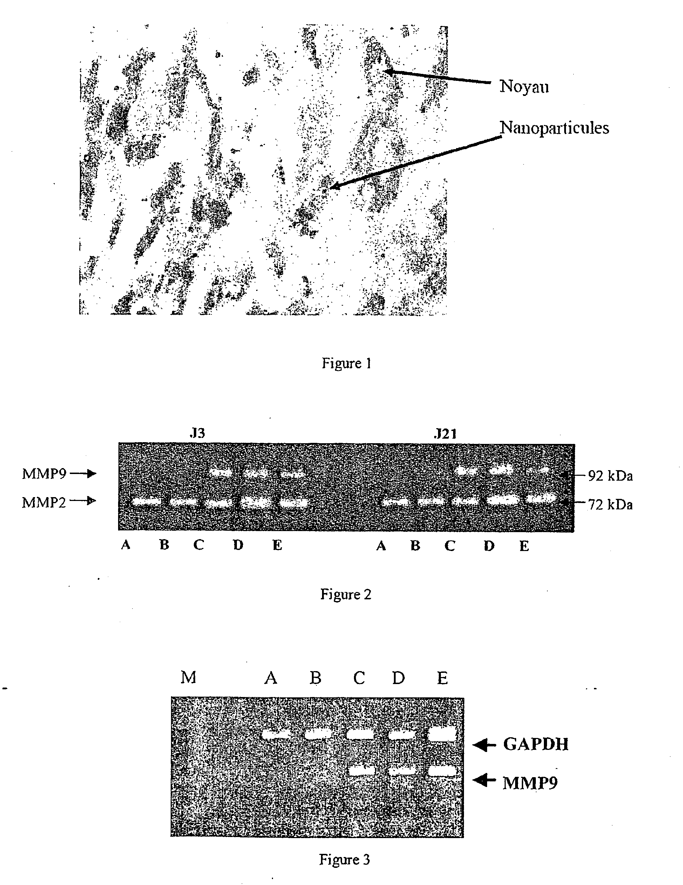

La qualité de marquage des fibroblastes gingivaux par ces nanoparticules est contrôlé par coloration de Perl (bleu de prusse). Les nanoparticules sont absorbées et internalisées dans l'endosome des fibroblastes gingivaux, comme le montre la Figure 1.

Pour vérifier que le marquage ne modifie pas le phénotype des fibroblastes gingivaux, l'effet de l'incorporation des nanoparticules sur la sécrétion de la métalloprotéinase-2 matricielle (MMP2) et des cytokines IL-1β et TGFβ a été évalué à J1, J3 et J5 (jours de culture).

A J1, on observe chez les fibroblastes marqués une augmentation de la production de MMP2 et d'IL-1β qui reviennent au niveau normal à J3 et J5. Cette augmentation transitoire est probablement due au stress consécutif à l'incorporation de nanoparticules. En revanche, aucune modification de la production de TGFβ n'est observée pendant la période étudiée.

### Co-cultures de fibroblastes gingivaux et de cellules musculaires lisses

Des co-cultures de fibroblastes gingivaux (FG) marqués et de cellules musculaires lisses (CML) sont réalisées en gels de collagène selon la méthode décrite par GILLERY et al. (Experientia 45(1) : 98-101, 1989).

Brièvement, les cellules sont obtenues à partir de prélèvements gingivaux pour les fibroblastes gingivaux, et à partir de la média artérielle pour les cellules musculaires lisses. Les prélèvements (gencive et média artérielle) sont mis en primo-culture dans des boites de pétri dans du milieu DMEM/SVF20%. A confluence, les cellules sont trypsinisées et remises en culture dans un milieu DMEM/SVF10%. Après plusieurs passages dans ce milieu, les cellules sont mises en culture dans du collagène de type I (60 000 cellules dans 2 ml de collagène) pendant 3, 7, 14 ou 21 jours. Les milieux de culture (DMEM/SVF10%) dans lesquels les gels baignent sont changés toutes les semaines.

A titre de contrôle, des cultures de fibroblastes gingivaux et des cultures de cellules musculaires lisses sont effectuées séparément, dans les mêmes conditions.

### Sécrétion de MMP9 par les CML

La sécrétion de MMP9 est localisée par immunodétection à J3, et observée par microscopie optique (x160).

La MMP9 n'est pas détectée au niveau des fibroblastes : elle est détectée uniquement aux endroits correspondant à la localisation des CML dans le gel. Ces résultats corroborent de précédents travaux selon lesquels les fibroblastes n'expriment pas MMP9 (GOGLY et al., 1998, précité).

### Effets des interactions FG/CML sur l'activité de MMP2 et de MMP9 et sur la transcription de MMP9

### Evaluation de l'activité de MMP9 et de MMP2 par zymographie

20 µl d'échantillon de culture de FG et/ou de CML sont dilués au 1/2 dans du Tris 1M à pH 6,8 contenant 50% de glycérol et 0,4% de bleu de bromophénol, puis soumis à une électrophorèse en gel de polyacrylamide SDS 10% contenant 1 mg/ml d'α-caséine (Sigma Chemical) pendant 1 heure. Les gels sont lavés dans du Triton X-100 2,5% dilué dans de l'eau distillée, puis incubés dans 100 mM Tris-HCl, 5 mM CaCl₂, Brij-35 0,005%, NaN₃ 0,001%, à pH 7,4, pendant 36 heures, à 37°C. Les gels sont ensuite colorés avec du bleu de Coomassie 0,25% (Biorad, réf. G 250) (50% méthanol, 10% acide acétique) puis décolorés de manière appropriée (40% éthanol, 10% acide acétique). Les résultats sont présentés dans la Figure 2.
**A:** activité MMP2 pour 600 fibroblastes non marqués (contrôle) ;
**B:** activité MMP2 pour 600 fibroblastes marqués cultivés en gel de collagène ;
**C:** activités MMP2 et MMP9 pour 600 CML cultivés en gel de collagène ;
**D:** activités MMP2 et MMP9 pour 600 fibroblastes marqués cultivés + 600 CML cultivés séparément en gel de collagène (On ajoute les milieux des 2 cultures séparées, pour déterminer l'activité totale en l'absence d'interactions entre les deux types cellulaires) ;
**E:** activités MMP2 et MMP9 pour 600 fibroblastes marqués et 60 000 CML co-cultivés en gel de collagène.

Les résultats montrent que les fibroblastes gingivaux en culture n'expriment pas MMP9 (A et B) contrairement aux CMLs (C et D). En revanche, les fibroblastes inhibent l'activité de MMP9 sécrétée par les CML en co-culture (E). Les mêmes résultats sont observés à J3 et J21. En ce qui concerne la MMP2, on observe une diminution de l'activité dans les co-cultures (E) par rapport à ce qui pourrait être attendu théoriquement (D). Cette diminution est toutefois moins significative que celle de la MMP9.

### Evaluation de la transcription de MMP9 par RT PCR

Les ARN totaux (1 ou 2 µg) sont extraits des cultures ou des co-cultures de FG et de CML à J14, à l'aide du kit MMP-CytoXpress Multiplex PCR (BioSource International). Les ARNm obtenus sont rétro-transcrits à l'aide d'une transcriptase inverse, puis une PCR est réalisée à l'aide d'amorces spécifiques de MMP9 comme suit : une étape de dénaturation à 95°C pendant 1 min, 5 cycles de dénaturation à 94°C pendant 1 min et d'hybridation à 60°C pendant 4 min, 35-40 cycles de dénaturation à 94°C pendant 1 min et d'hybridation à 68°C pendant 2,5 min, et une étape finale à 70°C pendant 10 min suivie d'un refroidissement à 20°C. Le GAPDH, qui est transcrit de façon constitutive, est utilisé à titre de contrôle. Les produits de PCR obtenus sont soumis à une électrophorèse sur un gel d'agarose 2%. Le gel est examiné sous UV et photographié. Les résultats sont présentés dans la Figure 3.
**M:** marqueur de poids moléculaire ADN ;
**A:** culture de FG non marqués (1 µg ARN) ;
**B:** culture de FG marqués (1 µg ARN) ;
**C:** culture de CML (1 µg ARN) ;
**D:** co-culture CML et FG (1 µg ARN) ;
**E:** co-culture CML et FG (2 µg ARN).

Les résultats ne montrent aucune modification transcriptionnelle de MMP9 dans les cultures de CML (C) et les co-cultures CML et FG (D et E), alors qu'une diminution de l'activité de cette enzyme dans lesdites co-cultures a précédemment été mise en évidence par zymographie. Par conséquent, l'action des fibroblastes gingivaux se situe au niveau traductionnel ou post-traductionnel.

### Effets des interactions FG/CML sur la production de TIMP-1

L'inhibiteur de MMP9 (TIMP-1) est exprimé par les fibroblastes gingivaux, et inhibe la forme active de MMP9 en formant un complexe MMP9/TIMP-1 inactif.

Les interactions cellulaires sont étudiées au travers de l'expression de TIMP-1 à J3, J7, J14, J21 (jours de culture).

### Evaluation de la production de TIMP-1 par dot-blot

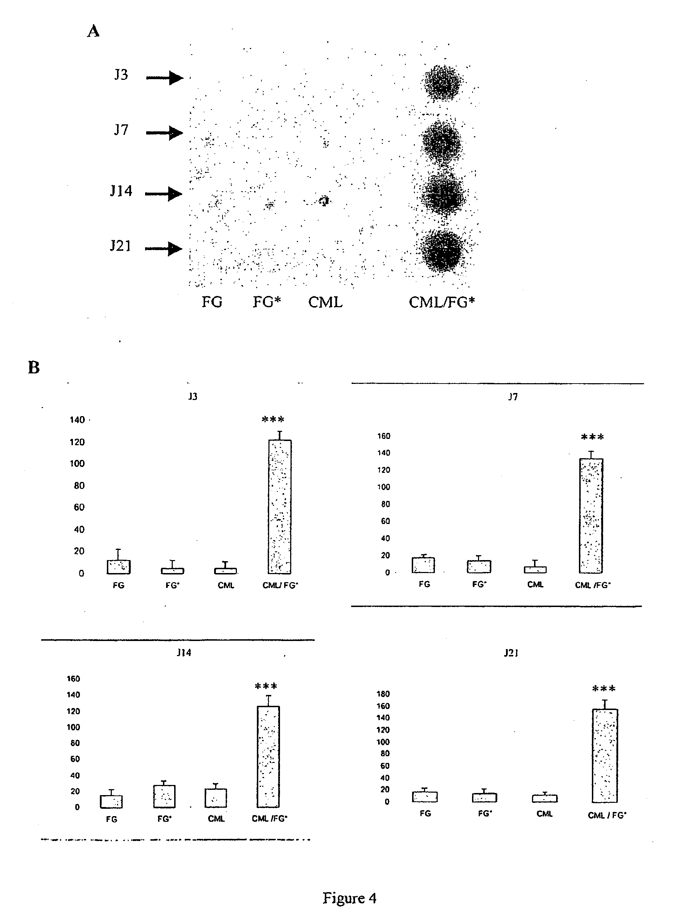

Des aliquots (1 ml) de surnageant de co-culture de FG et CML en gel de collagène sont amenés à un volume final de 100 µl dans du milieu DMEM, centrifugés à 10 000g pour éliminer les débris cellulaires, puis additionnés de 10 µl d'une solution de Tris-HCl 1M, NaCl 150 mM, pH 7,5. A titre de contrôle, des surnageants de culture de FG seuls ou de CML seuls sont préparés dans les mêmes conditions. Les échantillons (5 µl) sont déposés en trois exemplaires sur une membrane de nitrocellulose (Biorad, réf. 1 620 115). Les membranes sont incubées avec une solution bloquante à 1% (Boehringer, réf. 1 096 176) pendant 1 heure, à température ambiante, puis rincées 4 x 15 min dans du TBS-Tween (Tris 50 mM, NaCl 150 mM, Tween 20 0,1%, pH 7,5). Les membranes sont ensuite incubées avec un anticorps primaire polyclonal de chèvre anti-humain anti-TIMP-1 (1/500, R&D Systems, réf. AF970), toute une nuit, à température ambiante. L'anticorps primaire est spécifique des formes libres de TIMP-1. Après lavages dans du TBS-Tween (4 x 15 min), les blots sont incubés avec un anticorps secondaire marqué à la péroxydase (1/1000), pendant 1 heure, puis déposés dans une solution de révélation contenant du peroxyde d'hydrogène et du diacylhydrazide (Luminol, Boehringer, réf. 1 500 694) pendant 1 min, puis mis en contact avec un film photographique Kodak BIOMAX MR pendant une durée variant de plusieurs secondes à 10 min. Le film est alors révélé et fixé. Les multiples expositions sont examinées pour confirmer la linéarité des résultats. Les résultats sont présentés dans la Figure 4A.
Légende de la Figure 4A :
FG = culture de 10⁵ FG non marqués ;
FG* = culture de 10⁵ FG marqués ;
CML = culture de 10⁵ CML ;
CML/FG* = co-culture de 10⁵ FG marqués et de 10⁵ CML.

L'image du film est transmise à un ordinateur utilisant une caméra vidéo. Le logiciel (Imagenia 3000, station BIOCOM 200) évalue d'une part la densité des spots révélés sur les membranes, et d'autre part leur surface d'une manière semi-automatique par définition du contour. Les résultats sont présentés dans la Figure 4B.
Légende de la Figure 4B :
Abscisse
FG = culture de 10⁵ FG non-marqués
FG* = culture de 10⁵ FG marqués
CML = culture de 10⁵ CML
CML/FG* = co-culture de 10⁵ FG marqués et de 10⁵ CML Ordonnée = sécrétion de TIMP-1(pg/ml/10⁵ cellules)
*** = Student Fischer T test p<0,001

Les résultats montrent une forte augmentation de la sécrétion de TIMP-1 dans les co-cultures de FG et CML, en comparaison de sa sécrétion dans les cultures de FG seuls ou de CML seuls. Les mêmes résultats sont observés à J3, J7, J14 et J21.

### Evaluation de la transcription de TIMP-1 par RT PCR

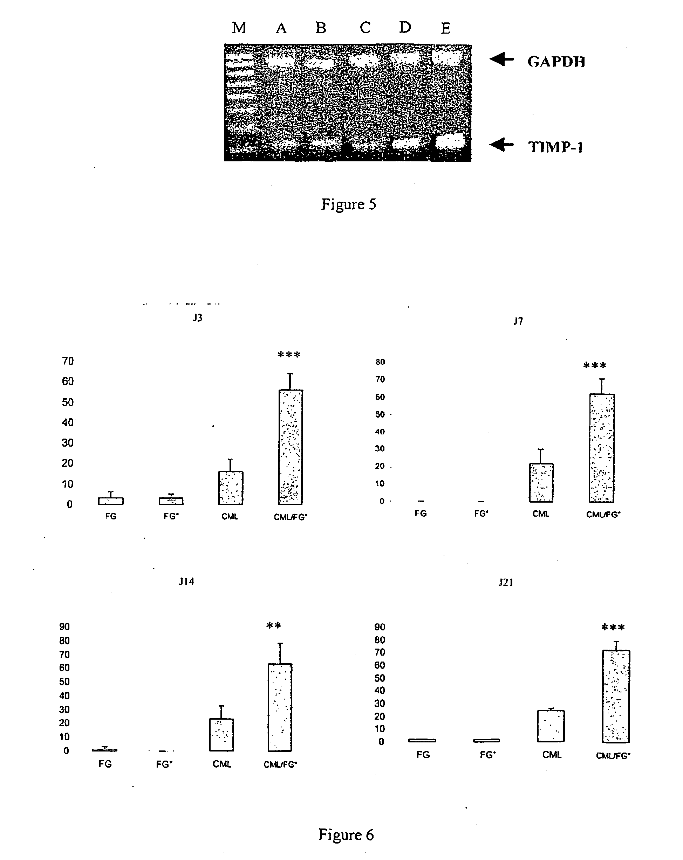

L'extraction des ARNm de TIMP-1 et la RT PCR sont réalisées comme précédemment décrit pour MMP9, à l'aide d'amorces spécifiques de TIMP-1. Les résultats sont présentés dans la Figure 5.
**M**: marqueur de poids moléculaire ADN ;
**A**: culture de FG non marqués (1 µg ARN) ;
**B**: culture de FG marqués (1 µg ARN) ;
**C**: culture de CML (1 µg ARN) ;
**D**: co-culture de CML et de FG (1 µg ARN) ;
**E**: co-culture de CML et de FG (2 µg ARN).

Les résultats montrent une augmentation de la transcription de TIMP-1 dans les co-cultures de CML et de FG (Figure 5, D et E), qui corrèle avec l'augmentation de la sécrétion de TIMP-1 (Figure 4).

### Evaluation de la production de complexes MMP9/TIMP-1 par test ELISA

Les interactions cellulaires sont étudiées au travers de la production des complexes MMP9/TIMP-1 à J3, J7, J14, J21 (jours de culture), par test ELISA à l'aide d'un kit DuoSet® ELISA Development (R&D Systems, réf. DY1449), selon les instructions du fabricant (anticorps spécifique des complexes MMP9/TIMP1. Les résultats sont présentés dans la Figure 6.
Légende de la Figure 6 :
Abscisse
FG = culture de 10⁵ FG non marqués
FG* =culture de 10⁵ FG marqués
CML =culture de 10⁵ CML
CML/FG* = co-culture de 10⁵ FG marqués et de 10⁵ CML
Ordonnée = sécrétion de MMP9/TIMP-1 (pg/ml/10⁵ cellules)
** = Student Fischer T test p<0,01
*** = Student Fischer T test p<0,001

Les résultats montrent que la quantité de complexes MMP9/TIMP-1 dosée par ELISA est augmentée (x4) dans les co-cultures de CML et de FG (CML/FG*). Les mêmes résultats sont obtenus à J3, J7, J14 et J21.

Il ressort de l'ensemble de ces résultats, que la diminution de l'activité de MMP9 par les CML co-cultivés avec les FG ne provient pas d'une diminution de sa synthèse mais d'une augmentation de la synthèse de son inhibiteur TIMP-1, et donc des complexes MMP9/TIMP-1 inactifs.

### Effets des co-cultures FG/CML sur la sécrétion de TGFβ

Les interactions cellulaires sont étudiées au travers de l'expression de la cytokine TGFβ à J3, J7, J14, J21/J28 (jours de culture).

### Evaluation de la production de TGFβ par test ELISA

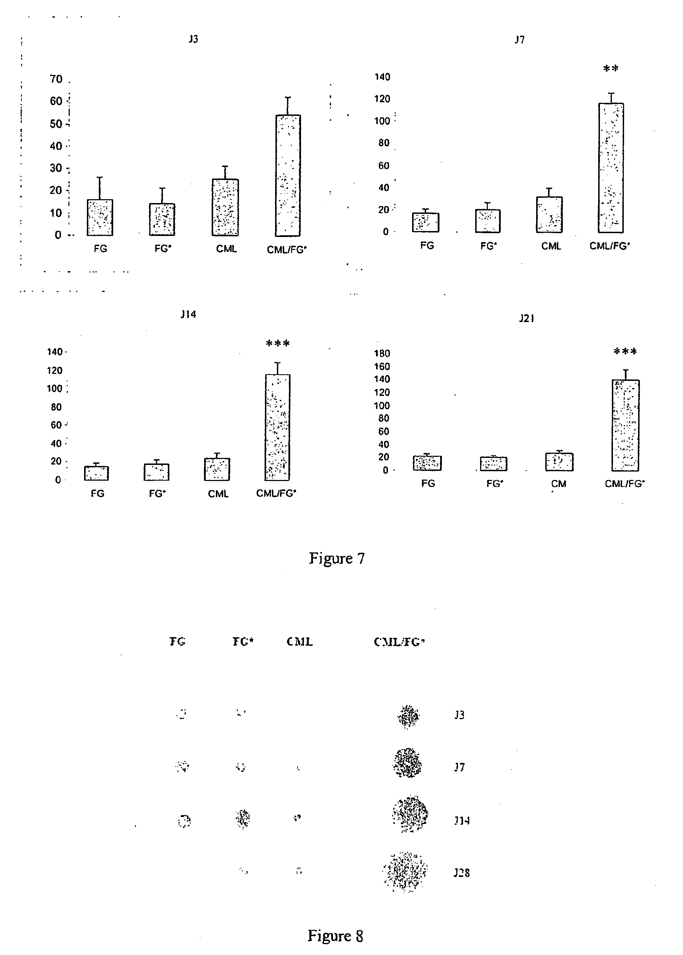

La production de TGFβ a été évaluée par test ELISA, à l'aide d'un kit DuoSet® ELISA Development (R&D Systems, réf. DY240), selon les instructions du fabricant. Les résultats de test ELISA à J3, J7, J14, J21 sont représentés dans la Figure 7.
Légende de la Figure 7 :
Abscisse
FG = culture de 10⁵ FG non -marqués ;
FG* =culture de 10⁵ FG marqués ;
CML =culture de 10⁵ CML ;
CML/FG* = co-culture de 10⁵ FG marqués et de 10⁵ CML.
Ordonnée = sécrétion de TGFβ (pg/ml/10⁵ cellules)
** = Student Fischer T test p<0,01
*** = Student Fischer T test p<0,001

### Evaluation de la production de TGFβ par dot-blot

La production de TGFβ a été évaluée par dot-blot, à l'aide d'un anticorps monoclonal de souris anti-TGFβ1 (R&D Systems, réf. MAB 240), et en utilisant le protocole décrit ci-dessus dans le cas de TIMP-1. Les résultats par dot-blot à J3, J7, J14, J28 sont représentés dans la Figure 8.
Légende de la Figure 8 :
FG = culture de 10⁵ FG non -marqués ;
FG* =culture de 10⁵ FG marqués ;
CML =culture de 10⁵ CML ;
CML/FG* = co-culture de 10⁵ FG marqués et de 10⁵ CML.

Les résultats montrent la potentialisation de la sécrétion de TGFβ par les cellules FG et CML en co-culture.

### EXEMPLE 2 : COMPARAISON DES EFFETS DES FIBROBLASTES GINGIVAUX AVEC CEUX DES FIBROBLASTES DERMIQUES OU ADVENTICIELS SUR LES ACTIVITES MMP2 OU MMP9 DES CML

Des co-cultures de fibroblastes dermiques (FD) ou adventiciels (FA) et de cellules musculaires lisses (CML) sont réalisées en gel de collagène, en utilisant le même protocole que celui décrit dans l'Exemple 1 ci-dessus pour les fibroblastes gingivaux.

Les cellules sont obtenues à partir de prélèvements cutanés pour les fibroblastes dermiques, de prélèvements de l'adventice pour les fibroblastes adventiciels, et à partir de prélèvements de la média artérielle pour les cellules musculaires lisses.

A titre de contrôle, des cultures de fibroblastes dermiques ou adventiciels et des cultures de cellules musculaires lisses sont effectuées séparément, dans les mêmes conditions.

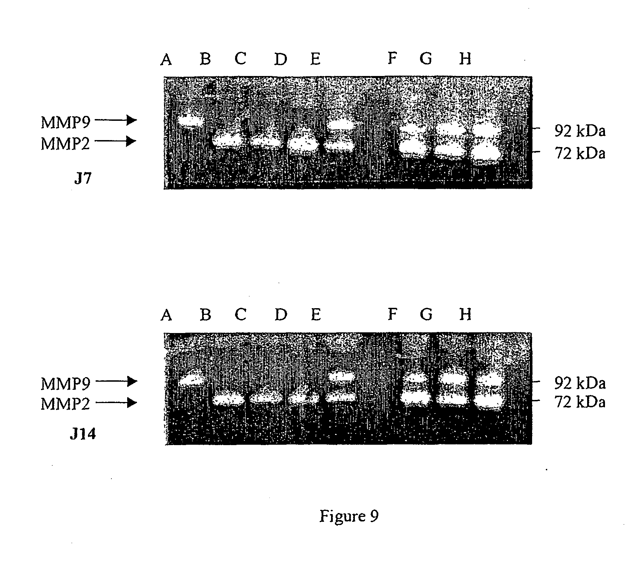

Les interactions cellulaires sont étudiées à J7 et J14 par détermination des activités MMP2 et MMP9, sécrétées dans le milieu de culture, par zymographie comme décrit dans l'Exemple 1 ci-dessus. En parallèle, 20 µl de MMP9 recombinante (R&D Systems, réf. 911-MP) sont soumis à la même électrophorèse afin de contrôler le type de MMP analysé. Les résultats sont présentés dans la Figure 9.
**A**: 20 µl contrôle (100 µg/ml) de MMP9 recombinante ;
**B**: activité MMP2 pour 600 FG cultivés en gel de collagène ;
**C**: activité MMP2 pour 600 FD cultivés en gel de collagène ;
**D**: activité MMP2 pour 600 FA cultivés en gel de collagène ;
**E:** activités MMP2 et MMP9 pour 600 CML cultivés en gel de collagène ;
**F:** activités MMP2 et MMP9 pour 600 CML et 600 FG co-cultivés en gel de collagène ;
**G**: activités MMP2 et MMP9 pour 600 CML et 600 FD co-cultivés en gel de collagène ;
**H**: activités MMP2 et MMP9 pour 600 CML et 600 FA co-cultivés en gel de collagène.

Les résultats montrent que, comme les fibroblastes gingivaux (B) et contrairement aux CML (E), les fibroblastes dermiques et adventiciels en culture n'expriment pas MMP9 (C et D). En revanche, les fibroblastes dermiques et adventiciels n'affectent pas l'activité MMP9 sécrétée par les CML en co-culture (G et H), contrairement aux fibroblastes gingivaux qui inhibent cette activité (F). Les mêmes résultats sont observés à J7 et J14. En ce qui concerne la MMP2, on n'observe aucune modification significative de l'activité des formes libres de cette enzyme. Les fibroblastes gingivaux, comme les fibroblastes dermiques ou adventiciels, ne semblent pas modifier la sécrétion de la MMP2 en co-culture avec les CML.

### EXEMPLE 3 : CULTURES ORGANOTYPIQUES D'ARTERES LESEES

La culture tridimensionnelle d'artères lésées en gel de collagène a été mise au point par les Inventeurs afin de fournir un modèle permettant l'analyse du remodelage artériel au cours du temps.

### Obtention des cultures

Des lésions de type athéroscléreuses sont induites chez 5 lapins blancs de Nouvelle-Zélande par la combinaison d'une dessication de l'air et d'une diète à forte teneur en cholestérol, comme décrit par LAFONT et al. (Circ. Res. 76(6) : 996-1002, 1995 ; Circulation 100(10) : 1109-1115, 1999). Après 4 semaines, la présence de lésions athéroscléreuses est confirmée par artériographie à l'intérieur des deux artères fémorales. Puis, des lésions iatrogènes sont produites sur les artères fémorales gauches à l'aide d'un cathéter à ballon pour angioplastie (3 insufflations à 6 atm pendant 60 sec).

24 heures après l'angioplastie, les lapins sont sacrifiés par injection intracardiaque de phénobarbital. Les artères sont collectées après dissection, et stockées dans du DMEM contenant du SVF 20%, à 4°C, pendant 3-10 heures, avant la mise en culture. De cette façon, 5 artères athéroscléreuses progressives (AAP) et 5 artères athéroscléreuses 24 heures après angioplastie (AAA) sont collectées.

Les artères sont rincées avec une solution de Hanks, puis disséquées de manière à préserver l'adventice et éliminer les tissus environnants. Enfin, chaque artère est décomposée en 4 segments de 5-7 mm.

Chaque segment est placé dans un erlenmeyer de 10 ml contenant 6 ml de milieu de culture (DMEM/SVF20%, antibiotiques et antifongiques usuels), 3,4 ml de collagène de rat de type I (Jacques Boy Institut, REIMS, France), et 600 µl de 0,1N NaOH filtré, et homogénéisé avec la solution. Enfin, le tout est transféré dans une boîte de culture, dans un incubateur à 37°C/5% CO₂. Chaque semaine, 1 ml de milieu de culture est ajouté au surnageant pour compenser l'évaporation.

A J3, J7, J14 ou J28 (jours de culture), les artères et leurs réseaux de collagène sont récupérés, rincés avec du PBS 1X, et fixés dans une solution de PBS/paraformaldéhyde 4% pendant 48 heures. Ils sont ensuite déshydratés dans de l'alcool 70°, 95° puis 100°, et finalement dans du toluène avant inclusion dans la paraffine. Les blocs de paraffine sont préparés en vue de la coupe.

Les surnageants de culture des 5 séries d'artères sont stockés à -80°C.

### Evaluation morphométrique des cultures

Des coupes de 7 µm d'épaisseur sont effectuées au microtome, puis colorées en utilisant trois protocoles spécifiques différents :
Protocole hémalun/éosine : après réhydratation, les coupes sont recouvertes avec de l'hémalun pendant 5 min avant différentiation avec de l'eau du robinet, puis avec de l'éosine pendant 1 min. Les préparations microscopiques sont enfin rincées avec de l'eau distillée avant déshydratation et assemblage final.
Protocole rouge Sirius : après réhydratation, les coupes sont recouvertes avec du rouge Sirius pendant 30 min. Les préparations microscopiques sont enfin rincées dans de l'eau distillée avant déshydratation et assemblage final.
Protocole catéchine(+)-fuchsine : après partielle réhydratation (stoppée à l'alcool 95°), les coupes sont immergées dans une solution colorante, à l'abri de la lumière, pendant 2 heures 1/2. Un rapide rinçage avec de l'alcool 95° (avec 2 gouttes d'acide chlorhydrique) est réalisé avant déshydratation et assemblage final.

### Numération cellulaire

Les coupes colorées à l'hémalun-éosine sont observées sous microscope couplé à un ordinateur (BIOCOM 200 station). Le comptage semi-automatique des cellules est rendu possible en pointant les noyaux. Les résultats sont donnés en nombre de cellules par unité de surface.

A l'intérieur des proliférations de l'intima (AAP) et de la néointima (AAA), le nombre de cellules par unité de surface diminue significativement au cours du temps. Cette réduction est très significative entre J3 et J7 (p<0,001), significative entre J7 et J14 (p<0,01) et plutôt significative entre J14 et J28 (p<0,05).

A l'intérieur de la média, le nombre de cellules musculaires lisses est relativement stable, à la fois dans le cas des AAP et des AAA.

A l'intérieur du réseau collagène, le nombre de cellules par unité de surface augmente. Cette augmentation est très significative entre J3 et J7 (p<0,001), significative entre J7 et J14 (p<0,01). Cette augmentation n'est plus significative entre J14 et J28 : un effet seuil semble intervenir à J28.

### Morphologie artérielle

Avec le même procédé de digitalisation de l'image, des mesures de l'épaisseur de la média, et des mesures de l'épaisseur des proliférations de l'intima (AAP) et de la néointima (AAA) sont réalisées. Les mesures sont prise 10-12 fois, dans 6 zones différentes à l'intérieur de chaque artère en culture.

Ces mesures ne montrent pas de modifications significatives au cours du temps.

### Quantification des composants de la matrice extracellulaire

### Composants du réseau collagène

Après digitalisation de l'image, le logiciel peut évaluer la surface relative du composant collagène (sur des coupes colorées au rouge Sirius, observées en lumière polarisée pour mettre en évidence les collagènes fibrillaires).

Les composants du réseau collagène sont évalués dans les proliférations de l'intima (AAP) et de la néointima (AAA). Dans les deux cas, une augmentation de la densité du réseau collagène est observée au cours du temps. En outre, les mesures étant réalisées sur des coupes observées en lumière polarisée, l'augmentation observée concerne le composant fibrillaire du réseau collagène.

### Composants du réseau élastique

Après digitalisation de l'image, le logiciel peut évaluer la surface relative des réseaux élastiques (sur des coupes colorées à la catéchine(+)-fuchsine).

La surface relative des composants du réseau élastique dans la média présente une réduction au cours du temps. Dans le cas des AAP, la réduction est significative entre J3 et J14 (p<0,01) et très significative entre J14 et J28. Dans le cas des AAA, cette réduction n'est significative qu'entre J14 et J28 (p<0,06). Cependant, les AAP ont initialement (à J3) un composant élastique plus significatif que les AAA. Mais à J28, les composants du réseau élastique sont sensiblement équivalents entre ces deux séries d'artères.

Enfin, dans les proliférations de l'intima et de la néointima, une augmentation des composants du réseau élastique est observée au cours du temps. Cette augmentation est très significative entre J7 et J28 pour les AAP, et entre J14 et J28 pour les AAA (p<0,001).

### Fragmentation des fibres élastiques

Les coupes colorées à l'hémalun-éosine sont observées par microscopie électronique sous UV. Après photographie des coupes et étude informatique des photos, la longueur des fibres élastiques entre deux points de fragmentation est mesurée. Cette longueur reflète l'état de fragmentation : plus la longueur des fibres est courte, plus la fragmentation est significative.

A l'intérieur des AAP, les laminas élastiques sont bien organisées, parallèles entre elles et concentriques à J3. La fragmentation des fibres est alors très modérée. Cependant, la longueur des fibres élastiques diminue au cours du temps. Cette réduction est très significative entre J3 et J7 et J14 et J28 (p<0,001). Ceci reflète la fragmentation progressive des fibres élastiques, qui apparaissent désorganisées et fragmentées à J28.

A l'intérieur des AAA, les lamina élastiques sont désorganisées et fragmentées dès les premiers jours de culture. Cette fragmentation à J3 est similaire à ce qui est observée pour les AAP à J28. Cependant, ce phénomène de fragmentation continue à se développer au cours du temps et de manière aussi significative que dans le cas des AAP. Dans ce modèle, le résultat de microscopie UV montre que la longueur des fibres élastiques dans les artères lésées diminue de 466 µm à J3 à 87 µm à J28.

L'ensemble des analyses morphométriques montre le maintien de la cohérence tissulaire artérielle en culture jusqu'à la fin de la période d'expérimentation. En outre, les phénomènes dynamiques de remodelage cellulaire et matriciel observés *in vitro* correspondent aux données établies *in vivo*, à la fois dans les situations inflammatoires violentes (AAA) ou lentes (AAP). Il apparait donc que les cultures organotypiques d'artères lésées en gel de collagène constituent un modèle d'anévrisme *ex-vivo* valable.

### EXEMPLE 4 : EVALUATION DE LA SECRETION D'ENZYMES CLES DU REMODELAGE ARTéRIEL PAR LES CULTURES ORGANOTYPIQUES D'ARTERES LESEES

### Sécrétion de MMP-1, -2 et -3, TIMP-1 et -2

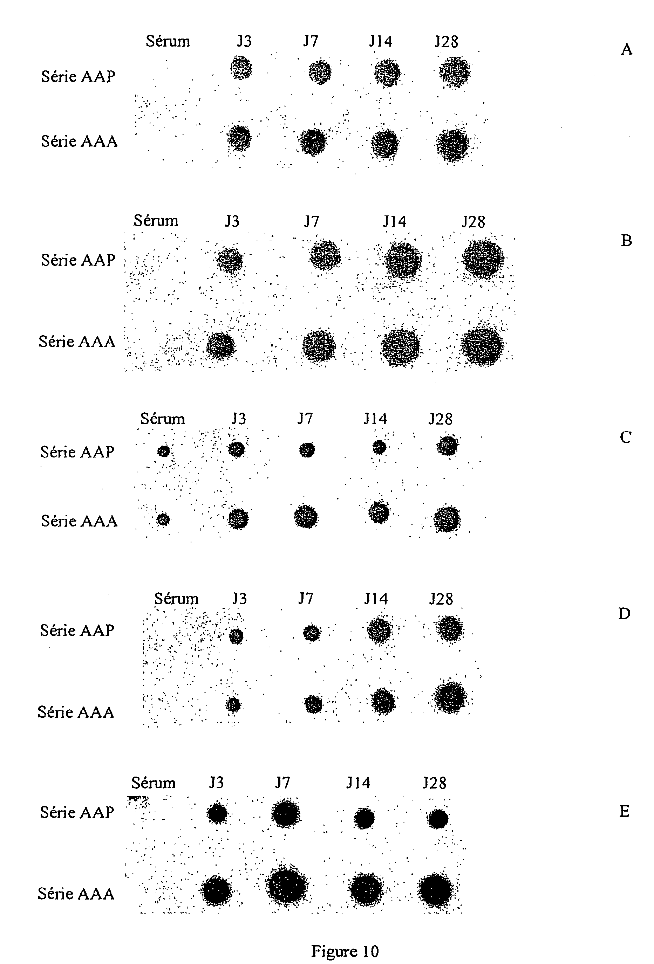

L'expression de MMP1, MMP2, MMP3, TIMP-1 et TIMP-2 sécrétées dans le milieu de culture organotypique d'artères AAA et AAP, est analysée par dot-blot comme décrit dans l'Exemple 1 ci-dessus, à l'aide d'anticorps humains dirigés contre ces MMPs et TIMPs (Valbiotech). A titre de contrôle, la présence des ces MMPs et TIMPs est également recherchée dans le sérum de culture (SVF). Les résultats sont présentés dans la Figure 10.
A: expression de MMP1
B: expression de MMP3
C: expression de MMP2
D: expression de TIMP-1
E: expression de TIMP-2

Entre J3 et J28, les résultats montrent :
- une augmentation significative de l'expression de MMP-1 (A) et MMP-3 (B), avec une concentration significativement plus importante dans les cultures AAA que dans les cultures AAP. MMP1 et MMP3 sont absentes du sérum de culture ;
- une augmentation de l'expression de MMP2 (C) au cours de la première semaine (J7) et une diminution au cours de la seconde semaine (J14), puis une nouvelle augmentation qui atteint son maximum à J28. Le même phénomène est observé dans les deux types de cultures d'artères, bien que MMP2 reste largement plus exprimée après angioplastie (AAA) qu'à l'intérieur des cultures AAP. En outre, les concentrations en MMP2 sont très significativement plus faibles dans le sérum que dans les surnageants de culture ;
- une augmentation graduelle de l'expression de TIMP-1 (D) au cours des deux premières semaines, sans différence significative entre les deux séries de cultures d'artères. En revanche, au cours des deux semaines suivantes, les cultures AAA présentent un pic de TIMP-1, alors que l'expression de l'enzyme stagne à l'intérieur des cultures AAP. TIMP-1 est absente du sérum de culture ;
- une augmentation de l'expression de TIMP-2 (E) la première semaine avant stagnation à des taux largement inférieurs à l'intérieur des cultures AAP. Après angioplastie, un pic d'expression de TIMP-2 est également observé la première semaine avant stagnation à des concentrations très élevées. En outre, TIMP-2 reste largement plus exprimée après angioplastie que dans les cultures AAP. TIMP-2 est absente du sérum de culture.

### Production de MMP9

L'expression de MMP2 et MMP9, sécrétées dans le milieu de culture organotypique d'artères AAA et AAP, est analysée.

### Evaluation de l'activité de MMP9 par zymographie-gélatine

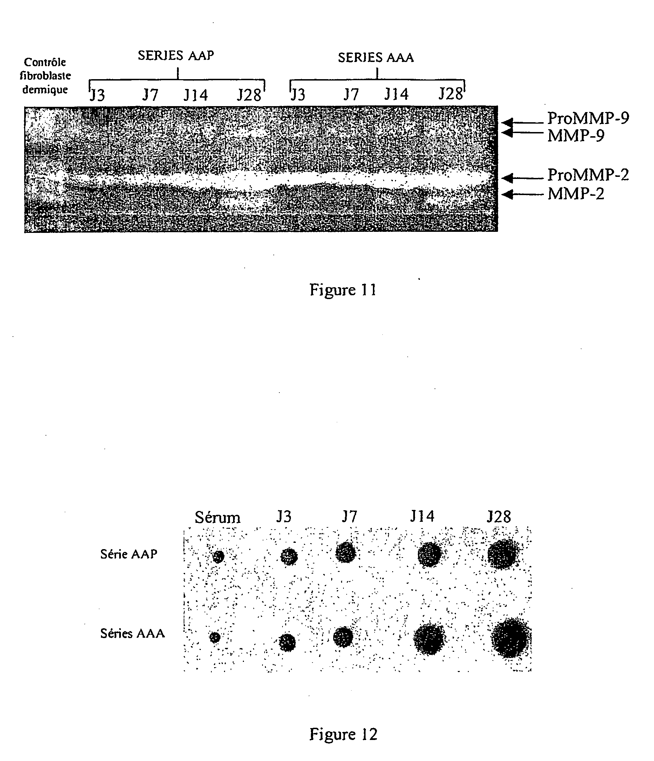

70 µl de surnageant de culture organotypique d'artères (AAP et AAA) sont dilués au 3/5 dans du Tris 1M à pH 6,8 contenant 50% de glycérol et 0,4% de bleu de bromophénol, puis soumis à une électrophorèse en gel de polyacrylamide SDS 10% contenant 1 mg/ml de gélatine de peau de porc (Sigma, réf. G2500) dans une solution tampon Laemmli, à 4°C, sous 80 volts dans le gel de concentration, puis 180 volts dans le gel de séparation. Après migration, les gels sont lavés dans du Triton X-100 2,5% (2 fois 30 min à température ambiante), puis incubés dans une solution tampon saline, à 37°C, pendant 48 heures. Les gels sont ensuite colorés avec du bleu de Coomassie 0,25% (Biorad, réf. R250) puis décolorés ; les bandes d'activité enzymatique apparaissent translucides, comme montré dans la Figure 11.

L'analyse par morphométrie informatisée rend possible la quantification des activités gélatinolytiques révélées par la zymographie. L'image du gel est transmise à l'ordinateur en utilisant une caméra vidéo. Le logiciel (Imagenia 3000, station BIOCOM 200) transforme l'intensité des bandes enzymatiques en niveau de gris : plus l'intensité de bande est élevée, plus le niveau de gris est élevé. L'absence de lyse correspond au niveau de gris 0, le maximum de lyse correspond au niveau de gris 255. La surface de bande est mesurée d'une manière semi-automatique par définition du contour. La quantification, en unité arbitraire U, est obtenue par le produit de la surface S de la bande et du niveau de gris.

Les résultats montrent une augmentation graduelle et significative de l'activité enzymatique de MMP2 et MMP9 entre J3 et J28, et qui apparaît plus significative après angioplastie (AAA) que dans les cultures AAP.

### Evaluation de l'expression de MMP9 par dot-blot

Le dot-blot est réalisé comme décrit dans l'Exemple 1 ci-dessus, à l'aide d'un anticorps humain dirigé contre MMP9 (Valbiotech). A titre de contrôle, la présence de MMP9 est également recherchée dans le sérum de culture (SVF). Les résultats sont présentés dans la Figure 12.

Les résultats montrent une augmentation très significative de MMP9 entre J3 et J28 à la fois dans le cas des cultures AAP et AAA (rapport de 1 à 4). En outre, à partir de J14, l'expression de MMP9 par les cultures AAA est significativement supérieure à celle des cultures AAP. Le sérum de culture contient une petite quantité de MMP9, mais cette quantité reste significativement inférieure par rapport aux concentrations mesurées dans les surnageants de cultures organotypiques d'artères.

### Sécrétion de cytokines

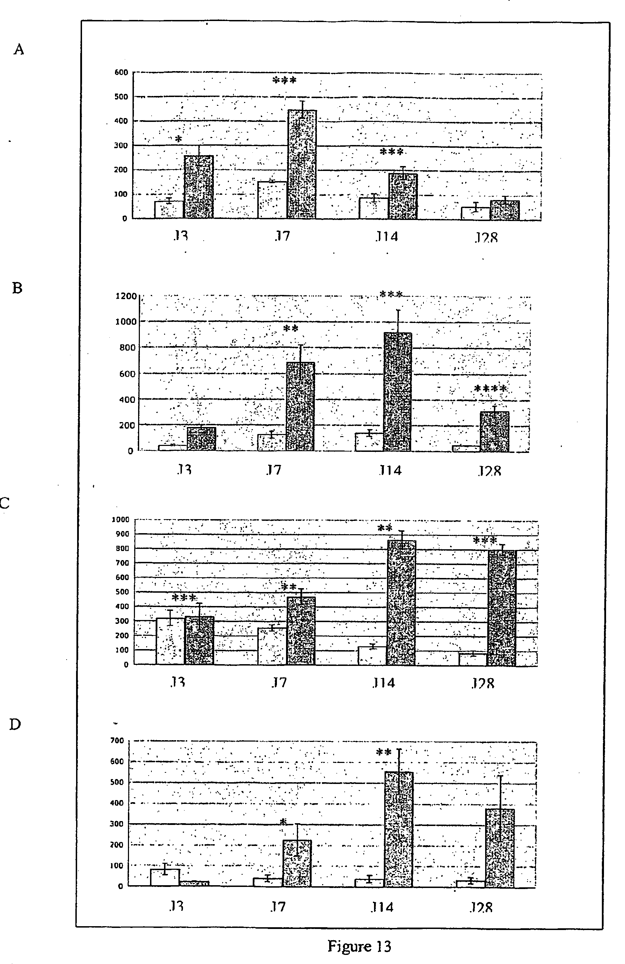

Les cytokines IL-1β, IL-4, IL-6, et TGFβ sont quantifiées dans le surnageant de cultures d'artères à J3, J7, J14 et J28, par test ELISA comme décrit à l'Exemple 1 ci-dessus, à l'aide respectivement des kits Quantikine® (R&D Systems, réf. DLB50), DuoSet® ELISA Development (R&D Systems, réf. DY204, réf. DY206 et DY240). Les valeurs moyennes obtenues sont transférées sous forme d'histogrammes et analysées statistiquement. Les résultats sont présentés dans la Figure 13.
Abscisse
**A**: quantification d'IL-1β ;
**B**: quantification d'IL-6 ;
**C**: quantification d'IL-4 ;
**D:** quantification de TGFβ ;
Ordonnée = pg/ml de cytokines secrétées par 24 heures
□ = culture d'artères AAP
■ = culture d'artères AAA (= après angioplastie)
* = Student Fischer T test p<0,05
** = Student Fischer T test p<0,02
*** = Student Fischer T test p<0,01
**** = Student Fischer T test p<0,001

Les résultats montrent :
- un pic de sécrétion d'IL-1β à J7, avant une brutale diminution (A). Ce phénomène est 4 fois plus important après angioplastie qu'à l'intérieur des cultures AAP ;
- un pic de sécrétion d'IL-6 différé (B) en comparaison de celui d'IL-1β, mais des rapports AAP/AAA très similaires à ceux d'IL-1β. La coordination fonctionnelle entre IL-1β et IL-6 est ainsi de nouveau confirmée par la corrélation de leur profil de sécrétion ;
- à partir de valeurs identiques à J3, une diminution significative des taux d'IL-4 (C) à l'intérieur des cultures AAP (taux :3) et une augmentation d'une manière proportionnellement identique des taux d'IL-4 après angioplastie (taux x3) ;
- une diminution très significative de la sécrétion de TGFβ (D) à l'intérieur des cultures AAP (taux :2), mais une augmentation très importante après angioplastie (facteur 20 entre J3 et J14).

### Immunodétections indirectes de MMP9 et MMP3

Des coupes de 7 µm d'épaisseur de cultures AAA et AAP, à J3 et J28, sont réhydratées (deux bains de toluène suivis par des solutions d'alcool 100° et 95°), puis traitées pendant 2 min avec de la pepsine 0,2% (Sigma) diluée dans l'acide acétique (1/10). Les préparations microscopiques sont rincées dans 0,1M PBS, pH 7,2, pendant 10 min, puis dans du PBS-glycine 1%, pendant 30 min. Les deux rinçages sont réalisés dans du PBS 1X.

Les coupes sont alors recouvertes avec une solution bloquante (1% BSA, PBS-Tween 0,05%, et sérum de cheval 10%) pendant 30 min, à 37°C.

La solution bloquante est éliminée et remplacée par un anticorps primaire dilué dans une solution contenant de la BSA 1% et du PBS-Tween 0,05% : 1/50 anti-MMP3 ou anti-MMP9 [Oncogène (Merk Eurolab)]. Les coupes sont couvertes avec l'anticorps primaire, pendant une nuit, à température ambiante.

Les préparations microscopiques sont rincées 3x10 min dans du PBS 1X. Les coupes sont alors couvertes avec un anticorps secondaire biotinylé spécifique pendant 90 min (Kit Vector Vestatain^{®} (ABC kit) Biovalley, Elite PK-6102). Les préparations microscopiques sont rincées 3x dans du PBS 1X.

Les peroxydases endogènes sont bloquées par l'addition de péroxyde d'hydrogène sur les coupes pendant 20 min, à 37°C. Les préparations microscopiques sont rincées 2 fois dans du PBS 1X et 1 fois dans du PBS-NaCl 3%. Le complexe avidine-biotine est préparé et déposé sur les coupes pendant 45 min, à température ambiante. Les préparations microscopiques sont rincées 2 fois dans du PBS-NaCl 3%, puis dans 0,1M Tris-HCl, pH 7,6.

La révélation finale est réalisée avec du DAB (DiAminoBenzydine) sur chaque coupe.

Une coloration de contraste par hémalun est réalisée pour améliorer le contraste entre l'immuno-marquage des péroxydases et les corps cellulaires.

Les coupes sont finalement déshydratées par deux bains successifs d'alcool (95° et 100°) suivis de deux bains de toluène. Les préparations microscopiques sont montées à l'aide de DEPEX (GURR^{®}).

A l'intérieur des cultures AAP, MMP3 et MMP9 apparaissent de manière diffuse dans la média à J3 avant de se concentrer le long de la lamina élastique interne à J14, et d'envahir l'intima. A J3, le marquage de MMP3 est beaucoup plus intense que celui de MMP9 ; mais le phénomène opposé se développe graduellement et, à J28, le marquage de MMP9 est très intense dans la média et spécialement dans l'intima, alors qu'il reste modéré pour MMP3.

A l'intérieur des cultures AAA, la dispersion de MMP9 apparaît intensément le long de la lamina élastique interne à J3 avant de migrer dans l'aire moyenne de la néointima, pour finalement s'étendre d'une manière diffuse à l'intérieur de toute la média. La distribution de MMP3 présente une cinétique clairement plus modérée : à J3, sa présence est encore diffuse dans la média, et c'est seulement à J7 que la dispersion de MMP3 s'accumule le long de la lamina élastique interne, avant de migrer dans l'aire moyenne de la néointima à J14, et de s'étendre à toute la néointima à J28. Cependant, le marquage de MMP3 reste toujours moins intense que celui de MMP9.

Ces résultats d'immunohistochimie sur des préparations microscopiques de cultures d'artères entre J3 et J28, permettent de confirmer une corrélation entre l'augmentation de la fragmentation des fibres élastiques et l'augmentation de la sécrétion de MMP9.

### EXEMPLE 5 : EFFETS DE CO-CULTURES D'ARTERES ET DE FIBROBLASTES GINGIVAUX SUR LA PRODUCTION DE MMP9, TIMP-1 ET SUR LE RESEAU ELASTIQUE

### Obtention de co-cultures d'artères et de fibroblastes gingivaux

Des segments d'artères lésées de lapin (lapin athérosclérotique AAP) obtenues comme décrit à l'Exemple 3 ci-dessus, sont mis en culture en présence de fibroblaste gingivaux dans les conditions suivantes : 100 000 fibroblastes gingivaux autologues, prélevés chez un lapin 1 mois avant son sacrifice pour les co-cultures artères/FG, sont mélangés au gel de collagène avant sa polymérisation, puis le fragment artériel de 2 mm (environ 3 mg) est déposé au milieu du gel. Les techniques de culture sont décrites dans les Exemples 1 et 3 ci-dessus.

### Effets des interactions FG/artères sur l'expression de MMP9 et de TIMP-1

Les interactions cellulaires sont étudiées entre J3 et J21 (jours de culture) par l'expression de MMP9 et TIMP-1, sécrétées dans le milieu de co-culture d'artères et de FG. A titre de contrôle, l'expression de ces enzymes est également analysée dans des cultures de FG seuls et dans des cultures organotypiques d'artères, effectuées dans les mêmes conditions que celles décrites respectivement dans les Exemples 1 et 3 ci-dessus.

### Evaluation de l'activité de MMP2 et MMP9 par zymographie

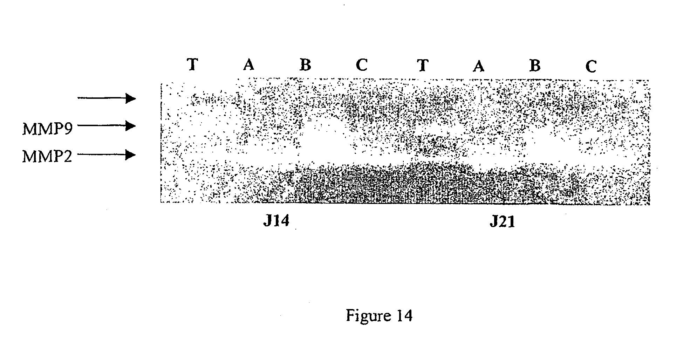

L'expression de MMP2 et MMP9 sécrétées dans le surnageant de co-cultures d'artères et de FG à J14 et J21, est analysée par zymographie-gélatine. Les résultats sont présentés dans la Figure 14.
**T**: 10 µl standard (100 µg/ml) de MMP9 recombinante (R&D Systems, réf. 911-MP) et de MMP2 recombinante (R&D Systems, réf. 902-MP) ;
**A**: culture cellulaire de 100 000 FG en gel de collagène ;
**B**: culture d'artères en gel de collagène ;
**C**: co-cultures d'artères et de 100 000 FG en gel de collagène.

Les résultats montrent que les fibroblastes gingivaux en culture n'expriment pas MMP9 (A et D) contrairement aux cultures organotypiques d'artères (B et E). En revanche, les fibroblastes gingivaux inhibent la sécrétion de MMP9 par les artères en co-culture (C et F). Les mêmes résultats sont observés à J14 et J21. En ce qui concerne la MMP2, on n'observe aucune modification significative de l'activité des formes libres de cette enzyme. Les fibroblastes gingivaux ne semblent pas modifier la sécrétion de la MMP2 en co-culture avec les artères.

### Evaluation de l'expression de MMP9 et TIMP-1 par dot-blot

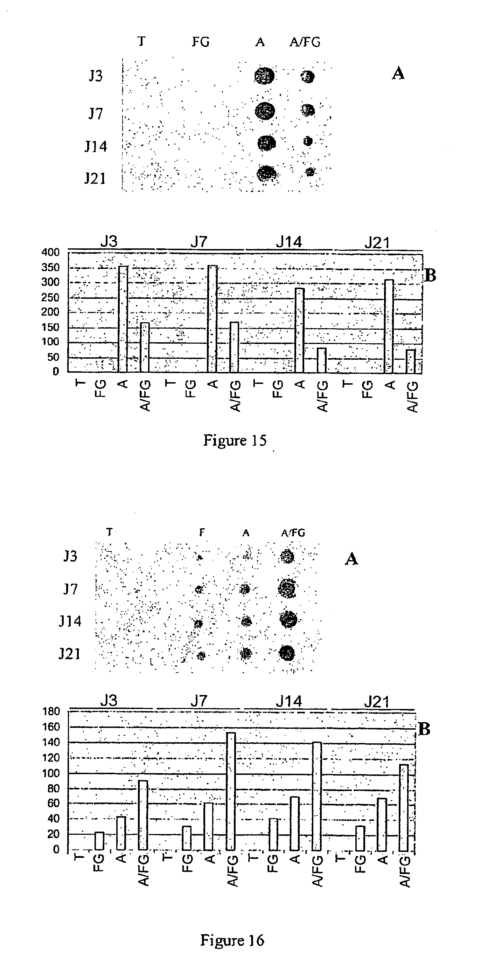

L'analyse par dot-blot du surnageant de co-cultures d'artères et de fibroblastes gingivaux entre J3 et J21 est réalisé comme décrit dans l'Exemple 1 ci-dessus, à l'aide d'anticorps anti-MMP9 et anti-TIMP-1. Les résultats de sécrétion de MMP9 et TIMP-1 sont présentés respectivement dans les Figures 15 (A = dot-blot, B = quantification du dot-blot) et 16 (A = dot-blot, B = quantification du dot-blot).
Abscisse
T = contrôle (10 pg de MMP9 recombinante, R&D Systems, réf. 911-MP) ;
FG = culture de 100 000 fibroblaste gingivaux en gel de collagène ;
A = culture organotypique d'artères en gel de collagène
A/FG = co-culture d'artères et de 100 000 fibroblastes gingivaux en gel de collagène.
Ordonnée = sécrétion de MMP9 (en pg).

Les résultats montrent que les fibroblastes gingivaux inhibent la sécrétion de MMP9 dans les co-cultures d'artères et de FG)= entre J3 et J21 (Figure 15). En parallèle, une forte augmentation de la sécrétion de TIMP-1 est observée dans les co-cultures A/FG en comparaison de celle détectée dans les cultures séparées FG et A (Figure 16). Les mêmes résultats sont obtenus à J3, J7, J14 et J21.

### Evaluation de la transcription de MMP9 et TIMP-1 par RT PCR

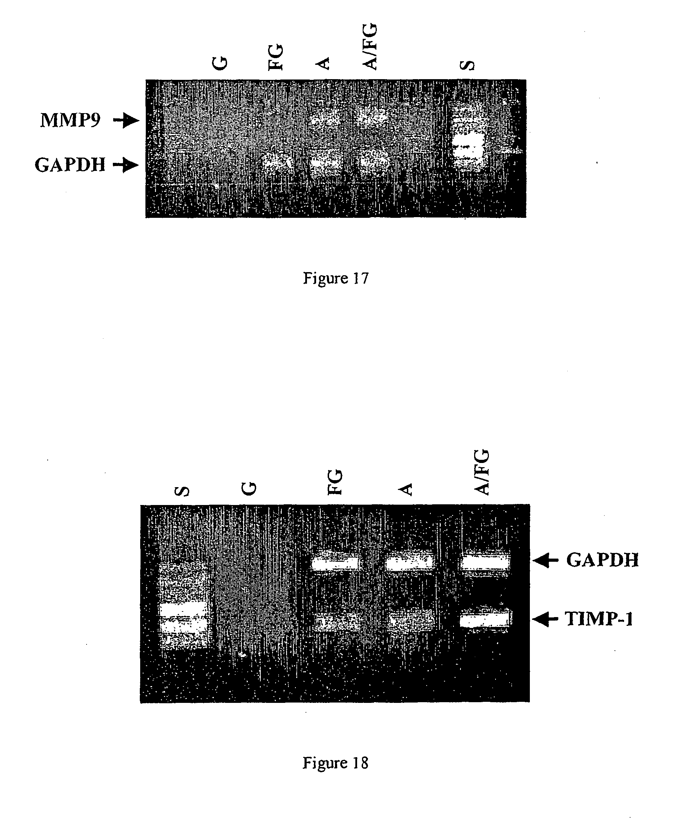

L'extraction des ARNm de MMP9 et de TIMP-1 à partir de co-cultures d'artères et de fibroblastes gingivaux à J14, et l'analyse par RT PCR, sont réalisées comme décrit dans l'Exemple 1 ci-dessus, à l'aide d'amorces spécifiques de MMP9 et de TIMP-1. Les résultats d'analyse transcriptionnelle de MMP9 et de TIMP-1 sont présentés dans les Figures 17 et 18, respectivement.
S: standard de poids moléculaire ADN (kit Multiplex PCR©, BioSource International) ;
G: gel de collagène seul ;
FG: culture cellulaire de FG (1 µg ARN) ;
A: culture organotypique d'artères (1 µg ARN) ;
A/FG: co-culture d'artères et de FG (1 µg ARN).

Les résultats ne montrent aucune modification transcriptionnelle de MMP9 dans les cultures d'artères et les co-cultures d'artères et de FG (Figure 17). En revanche, une augmentation de la transcription de TIMP-1 est observée dans les co-cultures d'artères et de FG en comparaison de celle observée dans les cultures de FG ou d'artères (Figure 18), qui corrèle avec l'augmentation de la sécrétion de TIMP-1 (Figure 16).

### Quantification des complexes MMP9/TIMP-1

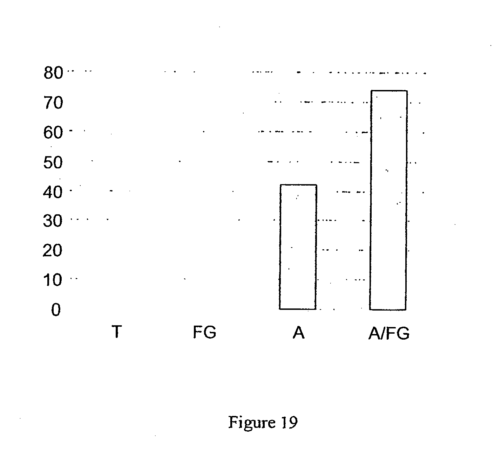

La quantification de l'expression de complexes MMP9/TIMP-1 dans les co-cultures d'artères et de fibroblastes gingivaux à J14, est réalisée par test ELISA comme décrit dans l'Exemple 1 ci-dessus. Les résultats sont présentés dans la Figure 19.
Abscisse
T = contrôle (gel de collagène seul)
FG = culture de 100 000 fibroblastes gingivaux en gel de collagène)
A = culture organotypique d'artères en gel de collagène
A/FG = co-culture d'artères et de fibroblastes gingivaux
Ordonnée = sécrétion de complexes MMP9/TIMP-1 (pg/ml).

Les résultats montrent que la quantité de complexes MMP9/TIMP-1 est augmentée dans les co-cultures d'artères et de FG (x 1,8).

L'ensemble des résultats biochimiques montre que les fibroblaste gingivaux en co-culture avec des artères inhibe l'activité de MMP9 sécrétée par CMLs artérielles, non en diminuant la synthèse de l'enzyme mais en induisant une augmentation de la synthèse de TIMP-1 et la formation de complexes MMP9/TIMP-1 inactifs, quelque soit le temps de culture (de J3 à J21). Ces résultats *ex-vivo* confirment donc ceux observés *in vitro* (Exemple 1 ci-dessus).

### Analyse du réseau élastique

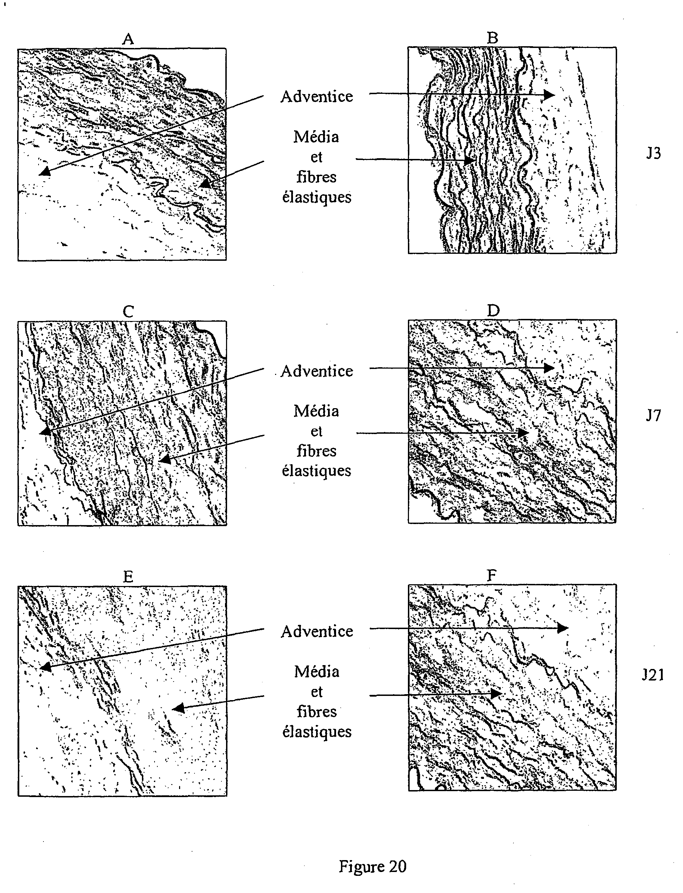

Des coupes de 7 µm provenant d'artères lésées co-cultivées avec des fibroblastes gingivaux pendant 3, 7 et 14 jours, sont observées sous microscope (x40) après coloration par l'orcéine. A titre de contrôle, des coupes d'artères cultivées seules comme décrit dans l'Exemple 3 ci-dessus sont analysées, dans les mêmes conditions. Les résultats sont présentés dans la Figure 20.
A J3
**A**: artère cultivée seule
**B**: artère co-cultivée avec des fibroblastes gingivaux
A J7
**C**: artère cultivée seule
**D**: artère co-cultivée avec des fibroblastes gingivaux
A J21
**E**: artère cultivée seule
**F**: artère co-cultivée avec des fibroblastes gingivaux

Dans les artères cultivées seules, on observe un raccourcissement et une disparition progressive des fibres élastiques au cours du temps (A, C et E), alors que dans celles co-cultivées avec des FG, le réseau élastique demeure dense (B, D et F). Les résultats montrent donc une protection du réseau élastique au cours du temps, quand les artères sont cultivées en présence de fibroblastes gingivaux.

Il ressort de cette étude, que la conservation de l'intégrité du réseau élastique de la média est corrélée avec une diminution de l'activité de MMP9 consécutive à une augmentation de TIMP-1. Le fibroblaste gingival apparaît donc comme une cellule pouvant modifier le remodelage vasculaire, en reproduisant dans l'artère son potentiel réparateur exprimé naturellement dans la gencive.

### EXEMPLE 6 : TRANSPLANTATION DE FIBROBLASTES GINGIVAUX HUMAINS IN VIVO DANS DES ARTERES DE LAPIN

L'objectif de cette étude est de déterminer la faisabilité de l'injection et la validation du marquage des fibroblastes par nanoparticules vérifiés par la coloration de Perl's. Les cellules humaines injectées chez le lapin permettent de visualiser par immunodétection HLA si les cellules colorées au Perl's sont bien les fibroblastes injectés. En effet les nanoparticules pourraient s'échapper de FG morts et marquer des CML de la média.

La transplantation de fibroblastes gingivaux humains est réalisée *in vivo* dans des artères de lapin dans deux modèles différents : un modèle d'artères normales et un modèle d'anévrisme (modèle élastase).

Les fibroblastes gingivaux humains sont marqués en culture monocouche par les nanoparticules ferro-magnétiques, comme décrit dans l'Exemple 1 ci-dessus puis trypsinisés.

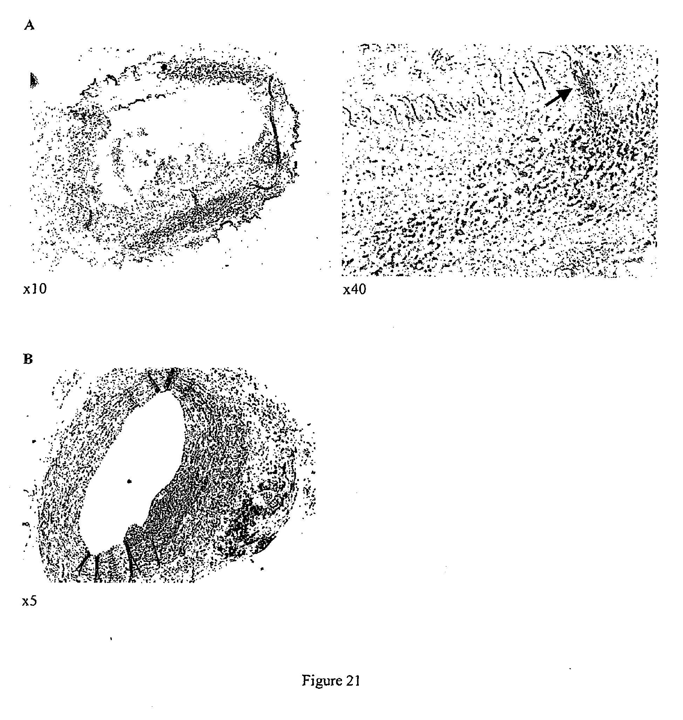

La procédure de transplantation est identique à celle décrite dans l'Exemple 6 ci-dessus. Les résultats sont présentés dans la Figure 21.
A : modèle d'artères normales (x10 et x40)
B : modèle d'anévrisme (x5)

Les résultats montrent qu'il existe une superposition du marquage HLA anti-humain et la coloration de Perl's (Figure 21A) ce qui confirme la validité de cette technique d'injection. Les cellules ont une morphologie normale, et on peut observer un site de pénétration de l'INFILTRATOR dans l'artère (cf. flèche Figure 21A, x40). La Figure 21B montre une injection de fibroblastes dans une artère anévrismale.

### EXEMPLE 7: TRANSPLANTATION DE FIBROBLASTES GINGIVAUX AUTOLOGUES IN VIVO DANS DES ARTERES DE LAPIN

Les fibroblastes gingivaux sont marqués en culture monocouche par les nanoparticules ferro-magnétiques, comme décrit dans l'Exemple 1 ci-dessus puis trypsinisés.

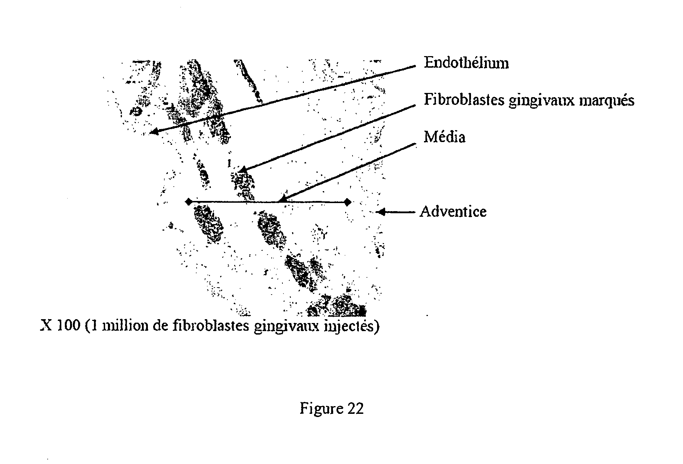

Ils sont injectés à différentes quantités (de 10⁵ à 5x10⁶ cellules) dans les artères iliaques de lapin à l'aide d'un cathéter INFILTRATOR (Boston Scientific) (TEIGER et al., Eur. Heart J. 18 : abstract suppl. 500, 1997). Les lapins sont sacrifiés 24h après l'injection et les lieux d'injections sont visualisés. Les résultats sont présentés sur la Figure 22.

A fort grossissement (X 100) la coloration de Perl permet de voir les nanoparticules sur les coupes histologiques de 6 µm. Les fibroblastes injectés sont localisés dans la média, et présentent une morphologie normale.

## Revendications

1. Utilisation de fibroblastes gingivaux pour l'obtention d'une composition cellulaire destinée au traitement d'une pathologie du remodelage artériel.

2. Utilisation selon la revendication 1, **caractérisée en ce que** ladite pathologie est un anévrisme.

3. Utilisation selon la revendication 1, **caractérisée en ce que** ladite pathologie est une sténose ou resténose après angioplastie.

4. Utilisation selon la revendication 1, **caractérisée en ce que** ladite pathologie est une dissection aortique.

5. Utilisation selon la revendication 1, **caractérisée en ce que** ladite pathologie est une athérosclérose.

6. Utilisation selon une quelconque des revendications 1 à 5, **caractérisée en ce que** lesdits fibroblastes sont issus de tissu gingival préalablement prélevé chez le sujet auquel est destiné le traitement.

## Claims

1. Use of gingival fibroblasts for obtaining a cell composition intended for the treatment of a pathological condition of arterial remodelling.

2. Use according to claim 1, **characterised in that** the said pathological condition is an aneurysm.

3. Use according to claim 1, **characterised in that** the said pathological condition is a stenosis or restenosis after angioplasty.

4. Use according to claim 1, **characterised in that** the said pathological condition is an aortic dissection.

5. Use according to claim 1, **characterised in that** the said pathological condition is atherosclerosis.

6. Use according to any one of claims 1 to 5, **characterised in that** the said fibroblasts are obtained from gingival tissue which has previously been taken from the patient for whom the treatment is intended.

## Patentansprüche

1. Verwendung von Zahnfleischfibroblasten zur Herstellung einer zellulären Zusammensetzung, die zur Behandlung einer Pathologie arterieller Remodelierung bestimmt ist.

2. Verwendung nach Anspruch 1, **dadurch gekennzeichnet, dass** die Pathologie ein Aneurysma ist.

3. Verwendung nach Anspruch 1, **dadurch gekennzeichnet, dass** die Pathologie eine Stenose oder eine Restenose nach einer Angioplastie ist.

4. Verwendung nach Anspruch 1, **dadurch gekennzeichnet, dass** die Pathologie eine Aortendissektion ist.

5. Verwendung nach Anspruch 1, **dadurch gekennzeichnet, dass** die Pathologie eine Arteriosklerose ist.

6. Verwendung nach einem der Ansprüche 1 bis 5, **dadurch gekennzeichnet, dass** die Fibroblasten aus Zahnfleischgewebe stammen, welches zuvor bei dem Subjekt entnommen wurde, für das die Behandlung bestimmt ist.
